Europäisches Patentamt

European Patent Office

Office européen des brevets

(11) Publication number: **0 251 134 B1**

# EUROPEAN PATENT SPECIFICATION

(45) Date of publication of patent specification: **19.11.92**    (51) Int. Cl.⁵: **A61K  31/725**

(21) Application number: **87108976.9**

(22) Date of filing: **23.06.87**

The file contains technical information submitted after the application was filed and not included in this specification

(54) **Compositions for preventing graft rejection.**

(30) Priority: **26.06.86 IL 79254**

(43) Date of publication of application:
**07.01.88 Bulletin  88/01**

(45) Publication of the grant of the patent:
**19.11.92 Bulletin  92/47**

(84) Designated Contracting States:
**AT BE CH DE ES FR GB GR IT LI LU NL SE**

(56) References cited:

**AUST. N.Z.J. MED., vol. 5, no. 6, 1975, pages 537-539; J.E. HORVATH et al.: "Low dose heparin and early kidney transplant function"**

**CARBOHYDRATE RESEARCH, vol. 145, January 1986, pages 267-277, Elsevier Science Publishers B.V., Amsterdam, NL; L. AYOTTE et al.: "N.M.R. spectroscopic observations related to the function of sulfate groups in heparin. calcium binding VS. biological activity"**

(73) Proprietor: **Hadassa Medical Organization P.O. Box 12000 Jerusalem(IL)**

Proprietor: **YEDA RESEARCH AND DEVELOP- MENT COMPANY, LTD. P.O. Box 26 Rehovot(IL)**

(72) Inventor: **Cohen, Irun 11 Hankin Street Rehovot(IL)**
Inventor: **Vlodavsky, Israel 502/18 Gilo Jerusalem(IL)**
Inventor: **Eldor, Amiram Burla Street Jerusalem(IL)**
Inventor: **Naparstek, Yaakov 17 Davidian Street Jerusalem(IL)**

(74) Representative: **Vossius & Partner Siebertstrasse 4 P.O. Box 86 07 67 W-8000 München 86(DE)**

Rank Xerox (UK) Business Services

METH. AND FIND. EXPTL. CLIN. PHARMACOL, vol. 4, no. 6, 1982, pages 359-363; M.L. TOIVONEN et al.: "Rat adjuvant arthritis as a model to test potential antirheumatic agents"

PHARMAZIE, vol. 28, no. 11/12, 1973, pages 744-748; H. BEKEMEIER et al.: "Zur Beeinflussung der Hyaluronidaseaktivität durch Antiphlogistica und andere Substanzen in vitro"

DIALOG INFORMATION SERVICES, file 72: embase 82-89, accession no. 5388930; S.N. CHIGIR: "Treatment of tuberculosis of the knee joint", & PROBL. TUBERK. (USSR), 1983, 61/3 (47-50)

AMERICAN JOURNAL OF PATHOLOGY, vol. 82, no. 1, January 1976, pages 25-42; R.W. COLMAN et al.: "Hyperacute renal allograft rejection in the primate"

TRANSPLANTATION, vol. 9, no. 1, January 1970, pages 1-7, The Williams & CO., US; A. McDONALD et al.: "Heparin and aspirin in the treatment of hyperacute rejection of renal allografts in presensitized dogs"

ANNUALS OF THE RHEUMATIC DISEASES, vol. 43, no. 4, 1984, pages 628-636; A.J. CRISP et al.: "Heparin modulates intracellular cyclic AMP in human trabecular bone cells and adherent rheumatoid synovial cells"

**Description**

There are provided pharmaceutical compositions adapted to prevent or diminish rejection of allografts, to prevent or alleviate autoimmune diseases comprising an effective dosage of heparin, or N-desulfated N-acetylated heparin or N-acetylated heparin, said dosage being of the order of from 1 to 10 percent of a dosage which results in a perceptible anti-coagulant effect.

There may be also other derivatives of, and modified forms of heparin, which result in the desired effect. Not all such derivatives are effective: for example, totally desulfated heparin is entirely without value for the intended purposes.

Along with its vital role in protecting the individual against foreign invaders, the immune system may attack the individual's own tissues, thereby producing autoimmune diseases. Another undesirable activity of the immune system is the rejection of critical transplanted organs. The ability of the immune system to produce autoimmune disease or reject allografts depends on the ability of lymphocytes, particularly activated T lymphocytes to enter the target organ or grafted tissue. Traffic to the target is by way of blood vessels and the activated T lymphocytes must be able to enter and exit through the vessel walls. Therefore, it is reasonable to suppose that the participation of T lymphocytes in autoimmune damage or graft rejection might be prevented by measures affecting their traffic.

In no previous studies were intact heparin or chemically modified heparins devoid of anti-coagulant activity shown to benefit autoimmune diseases or allograft rejection.

Subanticoagulant doses of 5000 units heparin per day showed no effect on allograft function or graft survival in humans (Horvath et al., Aust. N.Z.J. Med., Vol.5 (6), p.537-539, 1975).

It was discovered recently by us that T lymphocytes expressed a heparanase enzyme that specifically attacked the glycosaminoglycan moiety of the extracellular matrix secreted by endothelial cells that line blood vessels (Naparstek, Y., Cohen, I.R., Fuks, Z. and I. Vlodavsky. Activated T lymphocytes produce a matrix-degrading heparan sulphate endoglycosidase. Nature 310:241 (1984)). The presence of this enzyme was associated with the ablity of autoimmune T lymphocytes to penetrate blood vessel walls and to attack the brain in a model disease called experimental autoimmune encephalomyelitis.

Furthermore, it was found that the heparanase enzyme could be inhibited by heparin and some modified heparin molecules such as N-desulfated, N-acetylated heparin but not by others such as totally desulfated heparin (Table 1).

We therefore tested whether heparin or modified heparins administered to experimental animals might be used to treat autoimmune diseases or to prevent graft rejection.

1. N-desulfated, N-acetylated modified heparin, or a low dose of intact heparin has no anti-coagulant effect in rats. Table 2 shows that intact heparin at a dose of 2 mg per rat daily (10 mg/kg) caused an increase in the prothrombin time of recipient rats. In contrast, a dose of intact heparin of 0.02 mg (0.1 mg/kg) or 2 mg of N-desulfated,N-acetylated heparin (10 mg/kg) caused no anti-coagulant effect. Thus, the potential dangers of hemorrhage attendant upon the administration of 10 mg/kg of intact heparin could be avoided by using intact heparin at a low dose (0.1 mg/kg) or a chemically modified heparin devoid of anti-coagulant activity.

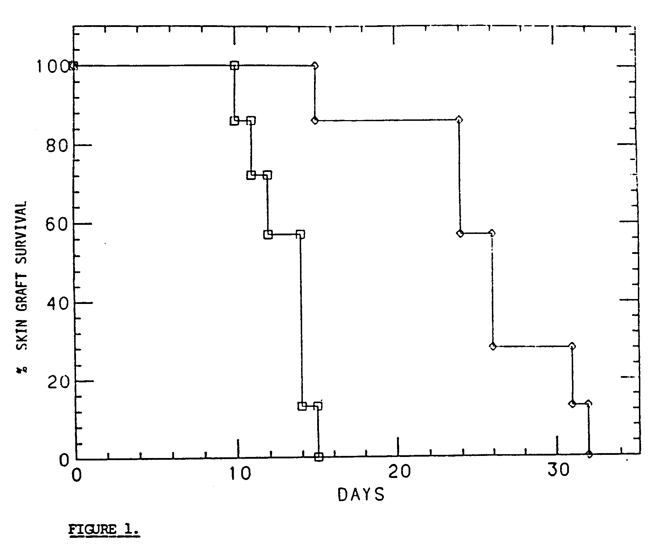

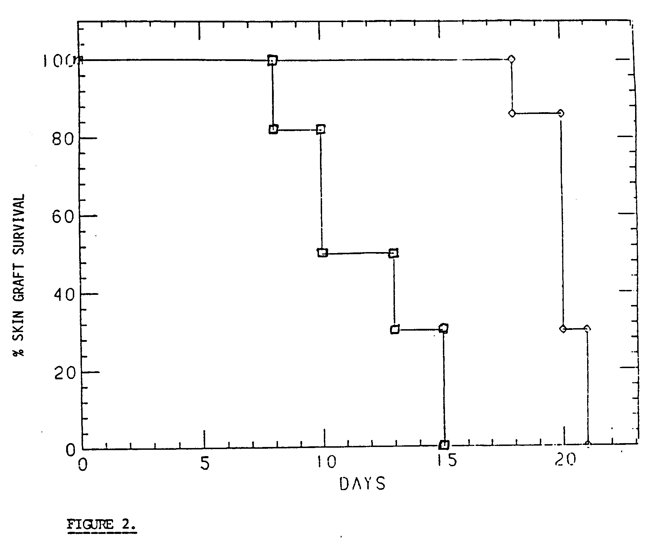

2. Modified or low dose heparin inhibits skin allograft rejection. Figure 1 shows the survival of SJL/J skin grafts on (BALB/cxC57BL/6)F1 mice. The median survival time of the skin grafts on control mice treated with saline was 14 days while that on mice treated with 0.05 mg/kg daily of heparin was 26 days with maximal survival to 32 days. Figure 2 shows that treatment with 10 mg/kg daily of N-desulfated, N-acetylated heparin increased the median survival of the allogeneic skin grafts from 10 to 20 days.

These results indicate that a low, sub-anti-coagulant dose of heparin or a modified, non-anti-coagulant heparin can significantly increase the survival time of allogeneic skin grafts on mice.

3. Modified or low dose heparin inhibits ability of anti-BP T lymphocytes to produce experimental autoimmune encephalomyelitis (EAE).

EAE is an experimental autoimmune disease with some features reminiscent of multiple sclerosis in humans The disease is caused by T lymphocytes immunized to the basic protein (BP) of the central nervous system myelin. To test the effect of heparins on the ability of T lymphocytes to cause autoimmune disease, we used T lymphocytes sensitized against BP, either as T cell lines (Cohen, I.R. Experimental autoimmune encephalomyelitis: Pathogenesis and prevention. In: Immunoregulatory Processes in Multiple Sclerosis and Experimental Allergic Encephalomyelitis. A.A. Vandenbark and J.C.M. Raus, eds. Elsevier Biomedical Res. Amsterdam. 7:91-125 (1985)) or as populations of lymph node cells from BP immunized rats. Table 3 shows that a sub-anti-coagulant dose of intact heparin (0.1 mg/kg/day) or a dose of modified heparin (N-desulfated, N-acetylated) devoid of anti-coagulant activity (10 mg/kg/day) was able to inhibit markedly the severity of EAE produced by the anti-BP T lymphocytes.

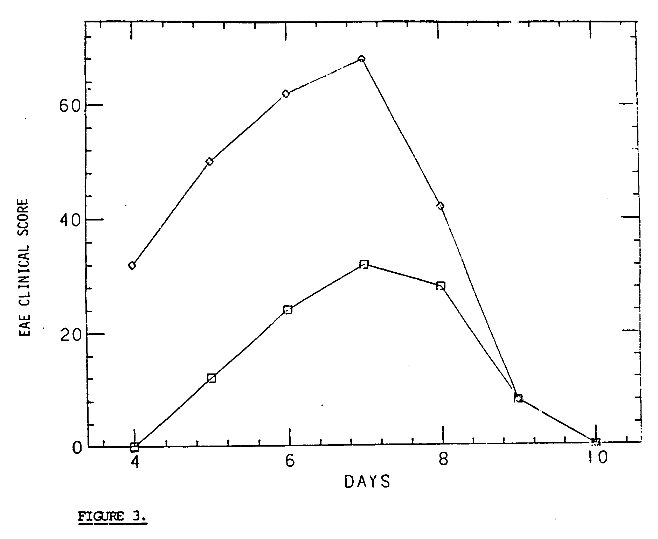

Figure 3 shows graphically the inhibition of EAE produced by treating rats with heparin (0.02 mg/rat/day; 0.1 mg/kg).

4. Modified or low dose heparin inhibits adjuvant arthritis.

Adjuvant arthritis is an experimental disease inducible in some strains of rats by immunizing them to antigens of Mycobacterium tuberculosis (Pearson, C.M. Development of arthritis, periarthritis and periostitis in rats given adjuvant. Proc. Soc. Exp. Biol. Med. 91:91 (1956)). The disease is thought to be a model of rheumatoid arthritis in humans (Pearson, C.M. Experimental models in rheumatoid disease. Arthritis Rheum. 7:80 (1964)). The arthritis appears to be caused by T lymphocytes that recognize an antigen of M. tuberculosis that is cross-reactive with cartilage (Cohen, I.R., J. Holoshitz, W. Van Eden, A. Frenkel. T lymphocytes illuminate pathogenesis and effect therapy of experimental arthritis. Arthritis Rheum. 29:841 (1985)).

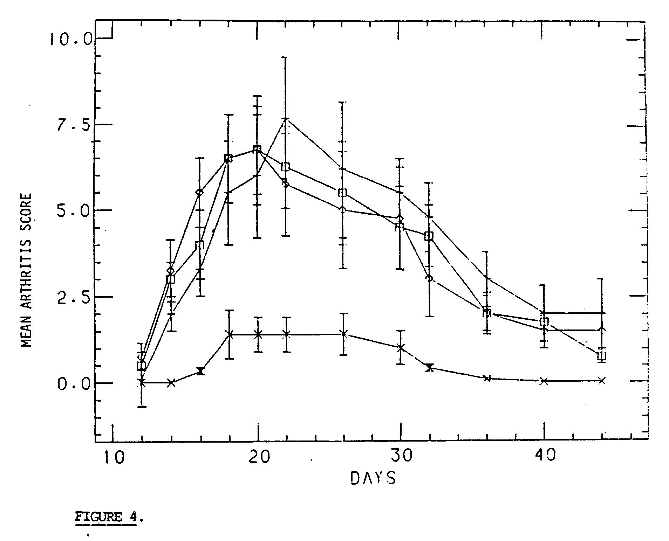

Table 4 shows that sub-anti-coagulant doses of heparin markedly inhibited adjuvant arthritis. A dose of heparin of 0.001 mg daily had a marginal effect on arthritis. Doses of 0.005 and 0.01 mg were more effective while a dose of 0.02 mg was maximally effective in inhibiting arthritis. However, the higher dose of 0.04 mg had no inhibitory effect. Thus the dose-response characteristics of treatment were very sharp; doubling the most effective dose led to total loss of activity. The sharpness of the dose response curve makes the beneficial effect of heparin on autoimmunity and graft rejection easy to miss and probably accounts for the oversight of other investigators in making our observation. Modified heparins such as N-desulfated, N-acetylated heparin also showed a similarly sharp dose-response curve with a maximum effect at 0.02 mg per rat (0.1 mg/kg). A higher dose (0.04 mg was ineffective (Figure 4).

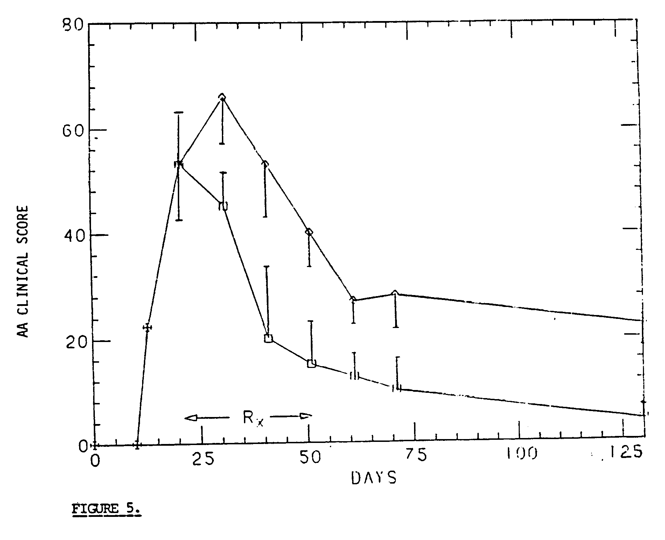

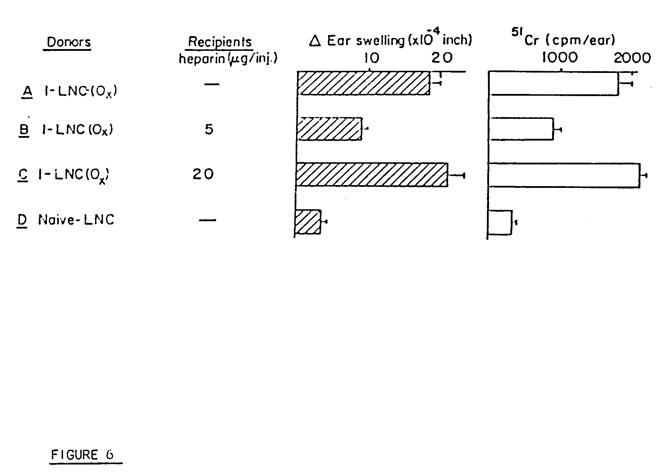

Figure 5 illustrates that modified heparin (N-desulfated, N-acetylated) at a dose of 0.1 mg/kg/day given from day 21 to 51 produced early remission of established adjuvant arthritis. Thus, treatment was effective even when the arthritis was already clinically severe. Histologic examination of the joints showed severe signs of inflammation in the control rats and healthy joints in the treated rats.

Table 5 tabulates the sources of commercially available heparin that were tested for their ability to produce long term inhibition (at day 60) of adjuvant arthritis subsequent to daily subcutaneous treatment for 5 days beginning on day 8 after induction of arthritis Heparin obtained from 3 of 4 sources were very effective while one source was only partially effective (Organon). Thus, a variety of sources can be used to obtain active material.

Table 6 illustrates the various modified heparins that were tested for their ability to produce long term inhibition of adjuvant arthritis at day 60 as described above. Total desulfated and N-desulfated heparins were not effective in treating arthritis. However, N-desulfated, N-acetylated and O-desulfated, N-acetylated heparins were as effective as was native heparin. As demonstrated in Table 2, the modified heparins had litle anti-coagulant activity. Thus inhibition of undesirable immunological reactions can be achieved with various preparations of heparin devoid of the danger of anti-coagulant activity.

5. Modified heparin inhibits entry into, and exit from, blood vessels of activated T lymphocytes.

To test the effect of modified heparin on T lymphocyte traffic, we labelled the lymphocytes with $^{51}$Cr and measured the uptake of the labeled lymphocytes from a subcutaneous site and their persistence in the blood. We found that the labeled lymphocytes persisted in the site of injection into the tail of rats or mice for 5-6 days in treated animals (N-desulfated, N-acetylated heparin; 0.05 mg/kg) While the labeled lymphocytes migrated from the site of injection within 1-2 days in control animals.

Furthermore, treatment with the modified heparin led to persistence of labeled lymphocytes in the blood for 4-5 days, while the untreated rats or mice cleared the labeled lymphocytes from the blood in 1 day. Thus, treatment with modified heparin inhibited the ability of T lymphocytes to enter the blood vessels, and once in the blood vessels, prevented the T lymphocytes from exiting. This can be attributed to inhibition of the heparanase enzyme activity needed to penetrate the extracellular matrix of the vessel wall For this reason, the T lymphocytes were less able to cause autoimmune disease or graft rejection.

The results indicate that an inhibitor of heparanase, such as heparin or N-desulfated, N-acetylated heparin, can be used to prevent autoimmunity and allograft rejection. The effective dose of heparin of the order of 0.1 mg/kg, is about 1% or less of that used to produce an anti-coagulant effect (10 mg/kg) and therefore prevention of undesirable immune reactions can be separated from anti-coagulation. N-desulfated, N-acetylated and O-desulfated, N-acetylated heparins are intrinsically devoid of anti-coagulant activity and can be used at a higher dose of the order of 10 mg/kg although lower doses (0.1 mg/kg) of these materials are more effective in preventing unwanted immune reactions. This dose is critical because, as shown in Table 4, 0.02 mg/rat (0.1 mg/kg) can be optimal in inhibiting disease while a higher dose, 0.04 mg/rat (0.2 mg/kg) can be ineffective. The same sensitivity of effect to dose was also observed with the modified heparins such as N-desulfated, N-acetylated.

This treatment is novel and avoids the generally toxic effects of immunosuppressive agents currently in use. We propose to patent the use of any heparin or modified heparin that inhibits T lymphocyte heparanase activity for the manufacture of a pharmaceutical composition for the treatment of autoimmune disease in humans, of the nervous system, joints, muscles, kidneys, liver, skin, digestive tract, liver, blood elements, endocrine organs or sex organs; or to prevent the rejection of allografts. This invention relates to the use of heparin, N-desulfated heparin or N-acetylated heparin for the preparation of pharmaceutical compositions effective against autoimmune diseases, for the alleviation of such diseases and for the treatment of autoimmune diseases in mammals, including humans, and for preventing or delaying allograft rejection, which compositions contain as active ingredient of from 1 per cent to 10 per cent of the dosage of heparin, N-desulfated heparin or N-acetylated heparin is required to obtain an anticoagulant effect.

Inhibition of DTH Type Skin Reactions

a. Assay system:

Mice were sensitized to 4 ethoxymethylene-2-phenyl oxazolone (OX) by painting their skins twice at 5 day intervals with about 0.1 ml of 3% OX in a vehicle of 4:1 acetone:olive oil (by volume). Their immunized draining lymph node cells (I-LNC) were then transfered ($5 \times 10^7$) to recipient mice intravenously. The ability of the I-LNC to reach the site of antigen and produce a DTH reaction was assayed by challenging the recipient mice 1 hr after I-LNC transfer with 0.02 ml of 0.5% OX painted on the ear. DTH was ascertained by measuring the thickness of the ears 24 hrs later with an engineers micrometer.

The ability of the transfered I-LNC to reach the ears was tested by labeling the I-LNC before transfer with radioactive $^{51}$Cr ($10^7$ cells/ml incubated with 0.1 mCi $^{51}$Cr sodium chromate and washed) and measuring the amount cpm reaching the ears at the time of DTH.

b. Low dose heparin inhibits migration to site of DTH.

Figure 1 shows that a dose of 5 u.g. daily of heparin prevents the I-LNC both from reaching the ear (decreased cpm) and from producing a DTH reaction (decreased ear swelling). A higher dose of heparin (20 $\mu$.g daily) did not inhibit either I-LNC migration to the ears or DTH reactivity.

c. Low dose heparin abrogates expression of heparanase in I-LNC

The above results indicated that treatment with the low-dose (5 $\mu$.g) of heparin suppressed the ability of DTH mediating I-LNC to enter blood vessels and to accumulate at the site of antigen deposit. To learn if these effects were associated with inhibition of endogenous heparanase, we treated mice with high (25 $\mu$.g) or low (5 $\mu$.g) doses of heparin, sensitized them to OX and tested their I-LNC for heparanase activity in vitro. Figure 2 shows that the I-LNC of mice treated with the low-dose (5 $\mu$.g) of heparin lacked heparanase activity. In contrast, the mice treated with the high (25 $\mu$.g) dose of heparin had heparanase activity that was similar to that of untreated control mice. Thus, treatment with 5 $\mu$.g of heparin in vivo caused a substantial decrease of enzyme activity in sensitized lymphocytes.

d. Low-dose heparin does not abrogate an in situ DTH reaction

If inhibition of heparanase and heparanase-dependent traffic is the major mechanism by which low dose heparin suppresses DTH, then one might be able to bypass the inhibition of DTH by bypassing the need for vascular traffic of sensitized T lymphocytes. Accordingly we treated recipient mice with an inhibitory dose (5 $\mu$.g) of heparin, and then injected the donor I-LNC directly into the ears, rather than intravenously. Table 1 illustrates that putting the sensitized lymphocytes in situ bypassed the inhibitory effect on the DTH of 5 u.g. of heparin. Thus low dose heparin treatment appeared to inhibit DTH only when the DTH-mediat lymphocytes had to make their way to the site of the antigen by way of the circulation.

e. Conclusions:

1. A low dose of heparin inhibits DTH reaction, as it does graft rejection and autoimmune diseases in experimental animals.

2. These effects are associated with a decrease in T lymphocyte heparanase and T lymphocyte migration to the site of the antigen.

2. Low dose heparin inhibits DTH reactions in humans

a. Healthy volunteers

6 young adult medical students were tested at 48 hrs for their spontaneous skin DTH reactions to tuberculin, tetanus toxoid, mumps antigen, or diphtheria toxoid and then treated with a single daily subcutaneous injection of 300-500 units of heparin. Two weeks later during continued heparin treatment, their DTH reactions were again measured and the positive reactions in each of the 6 were found to be markedly reduced. The heparin treatments were discontinued and 2 weeks later the DTH reactions were observed to return to their initial state of reactivity. Repeated administration of heparin again caused a marked decrement of DTH reactivity and stopping the treatment led to recovery of the original reactivity.

b. Patients suffering from multiple sclerosis

In preparation for a clinical trial of low dose heparin in multiple sclerosis, 12 patients were treated with daily doses of 300-1000 units of heparin, and similar to the healthy volunteers, all 12 had a decrease in DTH reactivity.

c. Low dose heparin induces improvement in rheumatoid arthritis.

As DTH reactions to self antigens are involved in autoimmune diseases, we have begun to test low dose heparin (about 300-500 units daily) in patients with rheumatoid arthritis. Three patients with severe arthritis were treated for 1 month and all 3 were improved; they felt better subjectively and they had a decrease in their clinical disability and arthritis as assessed by their physicians.

LEGENDS TO FIGURES:

FIGURE 1. Treatment with heparin (005 mg/kg) prevents rejection of skin allografts. Mice of hybrid strain (BALB/c x C57BL/6)F1 were grafted with skin from allogeneic SJL/J mice. The mice (20 per group) were treated daily with subcutaneous injections of saline (squares) or with heparin ("Leo", 0.05 mg/kg; diamonds) and scored for skin graft survival. Median survival for the control group was 10 days while that for the heparin treated group was 24 days.

FIGURE 2. Treatment with N-desulfated, N-acetylated heparin (10 mg/kg) prevents rejection of skin allografts. Mice were grafted as described in the legend to Figure 1 and treated daily with saline (squares) or N-desulfated, N-acetylated heparin (10 mg/kg; diamonds). The median survival of the skin allograft in the control groups was 10 days while that in the treated group was 20 days.

FIGURE 3. Treatment with heparin (0.1 mg/kg) inhibits EAE produced by autoimmune T lymphocytes. Beginning 1 day before inoculation with the T lymphocytes, the rats were injected daily with 0.02 mg of heparin subcutaneously (0.1 mg/kg; squares). Control rats were injected with saline (diamonds). EAE clinical score was estimated as tail weakness -25; paralysis of hind limbs -50; paralysis of all 4 limbs -75; moribund state -100.

FIGURE 4. Treatment of adjuvant arthritis using modified heparin (N-desulfated, N-acetylated) at various doses. Rats were immunized to induce adjuvant arthritis as described in the legend to Table 4. On day 9 the rats were inoculated subcutaneously once daily with N-desulfated, N-acetylated heparin at doses of 0 mg ( ), 0.001 mg (-), 0.02 mg (x) or 0.04 mg ( ). The dose of 0.02 mg caused a significant inhibition of arthritis.

FIGURE 5. Treatment with N-desulfated, N-acetylated heparin (0.1 mg/kg) induces remission of established adjuvant arthritis (AA). Twenty Lewis rats were inoculated with Mycobacteria tuberculosis to induce AA as described (3,11). Clinical arthritis was scored on a scale of 0 (no arthritis) to 100 (marked swelling, tenderness and redness of all 4 paws). On day 21, when all of the rats were suffering from marked arthritis, 10 were inoculated subcutaneously with saline (diamonds) and 10 were treated with N-desulfated, N-acetylated heparin (0.1 mg/kg) until day 51.

Figure 6

Left:

Low dose heparin inhibits adoptively transfered DTH.

I-LNC were obtained from OX sensitized mice (groups A-C) and naive LNC from unsensitized mice (group D). The LNC were transferred intravenously to naive recipients that were treated (groups B-C) or untreated (groups A,D) with heparin (5 $\mu$.g or 20 $\mu$.g) injected 18 hrs and 1 hr prior to cell transfer and 20 hrs after cell transfer. DTH ear swelling was elicited by OX at the time of cell transfer and measured 24 hrs later

Right:

Heparin inhibits cell migration to DTH challenge site.

The experiment was done as described in Figure 1 (left), except that I-LNC were radiolabeled with $^{51}$Cr prior to cell transfer into naive recipient mice. The accumulation of I-LNC in the OX challenged ears is indicated as the $^{51}$Cr (cpm/ear).

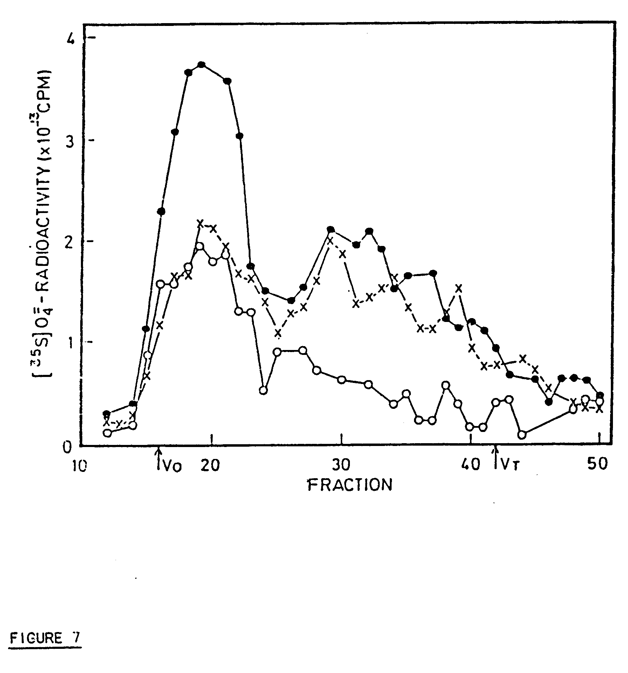

Figure 7 Heparin inhibits heparanase in vivo.

Mice were immunized with OX on days 0 and 5. Some of the mice were treated with heparin (5 $\mu$.g or 25 $\mu$.g per injection) 18 hrs before and 2, 10 and 20 hrs after the day 5 immunization with OX. On day 6, the I-LNC were removed and tested for heparanase activity by incubation for 48 hrs with labeled ECM. Heparan sulfate degradation products are shown for I-LNC from the following groups of mice: No heparin - 0; 25 $\mu$.g heparan - X; 5 $\mu$.g heparin - 0. The LNC of control mice not immunized to OX had no heparanase activity (not shown).

TABLE 1.

Inhibition of heparanase activity

| Test inhibitor | Inhibition of degradation of heparan sulfate by heparanase |
|---|---|
| Heparin: intact | yes |
| Heparin: totally desulfated | no |
| Heparin: N-desulfated N-acetylated | yes |

Heparanase activity was induced into the extracellular medium bathing activated T lymphocytes and tested by incubating the medium with extracellular matrix whose heparan sulfate was labeled with $^{35}$S as described (Naparstek, Y., Cohen, I.R., Fuks, Z. and I. Vlodavsky. Activated T lymphocytes produce a matrix-degrading heparan sulphate endoglycosidase. Nature 310:241 (1984)). Inhibition of heparanase activity was tested by adding various concentrations of heparin or modified heparins to the reaction mixture and measuring the effect on degradation of the labeled heparin-sulfate as described (Naparstek, Y., Cohen, I.R., Fuks, Z. and I. Vlodavsky.

Activated T lymphocytes produce a matrix-degrading heparan sulphate endoglycosidase. Nature 310:241 (1984)). Totally desulfated heparin and N-desulfated, N-acetylated heparin was prepared as described. (Ayotte, L., A.S. Perlin. NMR spectroscopic observations related to the function of sulfate groups in heparin. Calcium binding vs. biological activity. Carbohydrate Res. 145:267 (1986)).

TABLE 2.

Effect on prothrombin time of heparins

| Injected material | Dose (mg) | Prothrombin time (min) | Anti-coagulation |
|---|---|---|---|
| None | 0 | 19 | - |
| Heparin | 20 | 25 | yes |
| Heparin | 0.2 | 17 | no |
| Heparin: | | | |
| N-desulfated | | | |
| N-acetylated | 20 | 19 | no |

Lewis rats, 10 weeks old weighting 250 g, were injected subcutaneously with the indicated dose of heparin once daily for 2 days. The prothrombin time was then tested as described in the "Pathrombin Kit - OTX8" (Hoechst-Behring, Marburg, FRG).

TABLE 3.

Inhibition of experimental autoimmune encephalomyelitis (EAE) by treatment with a sub-ant-coagulant dose of intact heparin or with modified heparin (N-desulfated, N-acetylated).

| Agent | Dose (mg/kg) | Mediation of EAE | % incidence | Day of onset | Duration (days) | Clinical score |
|---|---|---|---|---|---|---|
| A. None | – | T cell line | 100 | 5.2 | 4.2 | 2.4 |
| Modified Heparin | 10 | | 50 | 6.4 | 1.8 | 0.8 |
| B. None | – | T cell line | 100 | 5.0 | 5.5 | 3.0 |
| Heparin | 0.1 | | 20 | 6.5 | 3.6 | 1.0 |
| C. None | – | Primed lymph node | 80 | 4.5 | 5.8 | 2.3 |
| Modified Heparin | 10 | | 0 | – | – | – |
| D. None | – | Primed lymph node | 100 | 4.0 | 5.3 | 3.0 |
| Heparin | 0.1 | | 75 | 6.3 | 4.0 | 1.5 |

EAE was produced by inoculating Lewis rats with a T cell line of anti-BP T lymphocytes ($10^6$ cells) with anti-BP primed lymph node cells ($10^7$ cells) intravenously (Cohen, I.R. Experimental autoimmune encephalomyelitis: Pathogenesis and prevention. In: Immunoregulatory Processes in Multiple Sclerosis and Experimental Allergic Encephalomyelitis. A.A. Vandenbark and J.C.M. Raus, eds. Elsevier Biomedical Res. Amsterdam. 7:91-125 (1985)) One day before inoculation and daily for 10 days, the rats received either saline or the heparins. The rats were observed for development of paralysis graded 1 for tail weakness; 2 for paralysis of hind limbs; 3 for paralysis of hind and forelimbs; and 4 for moribund state

TABLE 4.

Treatment of adjuvant arthritis by sub-anti-coagulant doses of heparin.

| Heparin | Adjuvant Arthritis | |
|---|---|---|
| dose | Duration | Maximum |
| (mg) | (days) | clinical |
| | | score |
| 0 | >20 | 10 |
| 0.04 | >20 | 10 |
| 0.02 | 8 | 2 |
| 0.01 | 15 | 5 |
| 0.005 | 16 | 4 |
| 0.001 | 20 | 6 |

Rats were immunized with M. tuberculosis (1 mg) in oil to induce adjuvant arthritis (Pearson, C.M. Development of arthritis, periarthritis and periostitis in rats given adjuvant. Proc. Soc. Exp. Bio. Med. 91:91 (1956)). On day 9 the rats were incubated subcutaneously once daily for 5 days with various doses of heparin and scored for the development of arthritis on a scale of 0-16 as described (Holoshitz, Y., Y. Naparstek, A. Ben-Nun, I.R. Cohen. Lines of lymphocytes mediate or vaccinate against autoimmune arthritis. Science 219:56 (1983)).

TABLE 5.

Sources of Heparin tested for inhibition of adjuvant arthritis.

| Heparin | Company | Arthritis Score (day 60) | Inhibition of Arthritis |
|---|---|---|---|
| Leo | Leo Pharmaceutical Ballerp, Denmark | 0 | yes |
| Sigma (bovine lung) | Sigma Chemical Co. St. Louis, MI, USA | 0 | yes |
| BDH | BDH Chemicals, Poole, England | 0 | yes |
| Thromboliquine | Organon Teknika, Boxtel, Holland | 2.5 | partial |
| Untreated | --- | 5 | - |

Adjuvant arthritis was induced in Lewis rats and the rats were treated with the indicated sources of heparin as described in the legend to Table 4. The mean arthritis score determined on day 60 was used to assay the efficacy of heparin treatment.

TABLE 6.

Modified heparins tested for inhibition of adjuvant arthritis.

| Heparin | Arthritis score (day 60) | Inhibition of arthritis |
|---|---|---|
| None | 6 | - |
| Intact | 0 | yes |
| N-desulfated N-acetylated | 0 | yes |
| O-desulfated N-acetylated | 0 | yes |
| Total desulfated | 5 | no |
| N-desulfated | 5 | no |

Adjuvant arthritis was induced in Lewis rats and the rats were treated with modified heparins as described in the legends to Tables 4 and 5. The heparins were modified as described (Ayotte, L., A.S. Perlin. NMR spectroscopic observations related to the function of sulfate groups in heparin. Calcium binding vs. biological activity. Carbohydrate Res. 145:267 (1986)).

12

TABLE 7.  This Table illustrates that heparin does not block transfer of DTH when I-LNC are directly injected into the site of antigen challenge

| Recipients of I-LNC sensitized to OX | | |
|---|---|---|
| Heparin treatment (5 µ.g) | OX Challenge | Ear swelling ($x10^{-4}$inch) |
| No | Yes | $22 \pm 3.4$ |
| Yes | Yes | $20.4 \pm 1.6$ |
| No | No | $9 \pm 1.0$ |
| Yes | No | $8 \pm 1.0$ |

I-LNC were obtained from BALB/c mice sensitized to OX 5 days earlier. The I-LNC were centrifuged, resuspended in RPMI medium and injected intradermally ($3x10^6$ cells/20 µ./l) into the dorsal surface of the ears of naive recipient mice. The ears were challenged with OX immediately after cell transfer. The magnitude of DTH ear swelling was determined 24 h later.

**Claims**
**Claims for the following Contracting States : BE, CH, DE, FR, GB, IT, LI, LU, NL, SE**

1. Use of heparin, N-desulfated, N-acetylated heparin or O-desulfated, N-acetylated heparin for the preparation of pharmaceutical compositions effective against autoimmune diseases, for the alleviation of such diseases and for the treatment of autoimmune diseases in mammals, including humans, and for preventing or delaying allograft rejection, which compositions contain as active ingredient of from 1 per cent to 10 per cent of the dosage of heparin, that is required to obtain an anticoagulant effect.

2. The use according to claim 1, wherein the dosage is of 0.01 to 0.2 mg/kg/day heparin.

3. The use according to claim 1, wherein the dosage is of 0.05 mg/kg/day N-desulfated, N-acetylated or O-desulfated, N-acetylated heparin.

4. The use according to claim 1, wherein the dosage of 0.02 mg/kg/day heparin is effective against arthritis.

**Claims for the following Contracting States : AT, ES, GR**

1. A process for the preparation of pharmaceutical compositions effective against autoimmune diseases, for the alleviation of such diseases and for the treatment of autoimmune diseases in mammals, including humans, and for preventing or delaying allograft rejection which comprises combining

13

heparin, N-desulfated, N-acetylated heparin or O-desulfated, N-acetylated heparin in an amount of from 1 per cent to 10 per cent of the dosage of heparin that is required to obtain an anticoagulant effect, with a suitable carrier.

2. The process according to claim 1, wherein the dosage is of 0.01 to 0.2 mg/kg/day heparin.

3. The process according to claim 1, wherein the dosage is of 0.05 mg/kg/day N-desulfated, N-acetylated heparin or O-desulfated, N-acetylated heparin.

4. The process according to claim 1, wherein the dosage of 0.02 mg/kg/day heparin is effective against arthritis.

**Patentansprüche**
**Patentansprüche für folgende Vertragsstaaten : BE, CH, DE, FR, GB, IT, LI, LU, NL, SE**

1. Verwendung von Heparin, N-desulfatiertem, N-acetyliertem Heparin oder O-desulfatiertem, N-acetyliertem Heparin zur Herstellung von Arzneimitteln, die gegen Autoimmunkrankheiten wirksam sind, zur Linderung dieser Krankheiten, zur Behandlung von Autoimmunkrankheiten bei Säugern, einschließlich Menschen, und zur Verhinderung oder Verzögerung der Abstoßung von Fremdtransplantat, wobei die Arzneimittel als Wirkstoff 1 bis 10 % einer Heparindosierung enthalten, die erforderlich ist, um eine koagulationshemmende Wirkung zu erzielen.

2. Verwendung gemäß Anspruch 1, wobei die Heparindosierung 0,01 bis 0,2 mg/kg/Tag beträgt.

3. Verwendung gemäß Anspruch 1, wobei die Dosierung 0,05 mg/kg/Tag N-desulfatiertes, N-acetyliertes oder O-desulfatiertes, N-acetyliertes Heparin beträgt.

4. Verwendung gemäß Anspruch 1, wobei eine Heparindosierung von 0,02 mg/kg/Tag gegen Arthritis wirksam ist.

**Patentansprüche für folgende Vertragsstaaten : AT, ES, GR**

1. Verfahren zur Herstellung von Arzneimitteln, die gegen Autoimmunkrankheiten wirksam sind, zur Linderung dieser Krankheiten, zur Behandlung von Autoimmunkrankheiten bei Säugern, einschließlich Menschen, und zur Verhinderung oder Verzögerung der Abstoßung von Fremdtransplantat, umfassend das Vereinigen von Heparin, N-desulfatiertem, N-acetyliertem Heparin oder O-desulfatiertem, N-acetyliertem Heparin in einer Menge von 1 bis 10 % einer Heparindosierung, die erforderlich ist, um eine koagulationshemmende Wirkung zu erzielen, mit einem geeigneten Träger.

2. Verfahren gemäß Anspruch 1, wobei die Heparindosierung 0,01 bis 0,2 mg/kg/Tag beträgt.

3. Verfahren gemäß Anspruch 1, wobei die Dosierung 0,05 mg/kg/Tag N-desulfatiertes, N-acetyliertes Heparin oder O-desulfatiertes, N-acetyliertes Heparin beträgt.

4. Verfahren gemäß Anspruch 1, wobei eine Heparindosierung von 0,02 mg/kg/Tag gegen Arthritis wirksam ist.

**Revendications**
**Revendications pour les Etats contractants suivants : BE, CH, DE, FR, GB, IT, LI, LU, NL, SE**

1. Utilisation d'héparine, d'héparine N-désulfatée et N-acétylée ou d'héparine O-désulfatée et N-acétylée pour la préparation de compositions pharmaceutiques efficaces contre les maladies autoimmunes, en vue du soulagement de telles maladies et du traitement des maladies autoimmunes chez le mammifère, y compris l'homme, et pour prévenir ou retarder le rejet de greffe allogène, lesdites compositions contenant en tant qu'ingrédient actif de 1 à 10 % du dosage de l'héparine requis pour obtenir un effet anticoagulant.

2. Utilisation selon la revendication 1, dans laquelle le dosage est de 0,01 à 0,2 mg/kg/jour d'héparine.

**3.** Utilisation selon la revendication 1, dans laquelle le dosage est de 0,05 mg/kg/jour d'héparine N-désulfatée et N-acétylée, ou d'héparine O-désulfatée et N-acétylée.

**4.** Utilisation selon la revendication 1, dans laquelle le dosage de 0,02 mg/kg/jour d'héparine est efficace contre l'arthrite.

**Revendications pour les Etats contractants suivants : AT, ES, GR**

**1.** Procédé pour la préparation de compositions pharmaceutiques efficaces contre les maladies autoimmunes, en vue du soulagement de telles maladies et du traitement des maladies autoimmunes chez le mammifère, y compris l'homme, et pour prévenir ou retarder le rejet de greffe allogène, ledit composé comprenant la combinaison d'héparine, d'héparine N-désulfatée et N-acétylée ou d'héparine O-désulfatée et N-acétylée selon une quantité de 1 à 10 % du dosage de l'héparine requis pour obtenir un effet anticoagulant avec un support adéquat.

**2.** Procédé selon la revendication 1, suivant lequel le dosage est de 0,01 à 0,2 mg/kg/jour d'héparine.

**3.** Procédé selon la revendication 1, suivant lequel le dosage est de 0,05 mg/kg/jour d'héparine N-désulfatée et N-acétylée, ou d'héparine O-désulfatée et N-acétylée.

**4.** Procédé selon la revendication 1, suivant lequel le dosage de 0,02 mg/kg/jour d'héparine est efficace contre l'arthrite.

FIGURE 1.

FIGURE 2.

FIGURE 3.

FIGURE 4.

FIGURE 5.

FIGURE 6

FIGURE 7